# EUROPEAN PATENT SPECIFICATION

(11) **EP 2 800 530 B1**
(45) Date of publication and mention of the grant of the patent: **19.07.2017**
(21) Application number: 13733624.4
(22) Date of filing: 07.01.2013
(51) Int. Cl.: A61N 7/02, A61N 7/00, A61B 17/225, A61B 8/12

(54) **HISTOTRIPSY THERAPY TRANSDUCER**
TRANSDUCER FÜR EINE HISTOTRIPSIE-THERAPIE
TRANSDUCTEUR DE THÉRAPIE PAR HISTOTRIPSIE

(30) Priority: 06.01.2012 US 201261583925 P
(43) Date of publication of application: 12.11.2014
(73) Proprietor: Histosonics, Inc., Ann Arbor, MI 48103 (US)
(72) Inventor: BERTOLINA, James, A., Ann Arbor, MI 48103 (US); RAKIC, Aleksandra, Ann Arbor, MI 48103 (US)
(74) Representative: J A Kemp
(86) International application number: PCT/US2013/020555
(87) International publication number: WO 2013/103975

(56) References cited:
- WO-A1-02/32506
- US-A- 5 443 069
- US-A- 5 601 526
- US-A1- 2003 112 922
- US-A1- 2006 264 760
- US-A1- 2008 214 964
- US-A1- 2009 112 098
- US-A1- 2009 227 874
- US-A1- 2011 054 315
- US-B1- 6 470 204
- US-B1- 6 470 204

## Description

### FIELD

This disclosure generally relates to applying therapeutic ultrasound to tissue. More specifically, this disclosure relates to transducer designs well suited to apply Histotripsy therapy to tissue.

### BACKGROUND

Histotripsy applies high intensity focused acoustic energy to homogenize cellular tissues. Histotripsy high intensity focused ultrasound is pulsed to induce acoustic cavitation in the target tissue. Acoustic cavitation occurs when rapid cycling from compression to rarefaction forms cavitation micro-bubbles which oscillate and collapse violently releasing tremendous energy as stresses and pressures. The resulting "bubble cloud" completely homogenizes the tissues. Such a device is described for example in US 5,443,069.

In order to generate an effective bubble cloud through layers of tissue from an external focused ultrasound transducer, pressures generated by the transducer must be very high. High pressures are necessary to overcome tissue attenuation and non-linearity. Compared to water, the average tissue attenuates the sound wave 0.8db/cm at 1MHz. Different attenuation in various tissues causes non-linearity. In order to generate a bubble cloud with sufficient energy to homogenize tissues at the target focal point, transducers must be capable of delivering very high pressure pulses through layers of attenuating soft tissues. In some clinical applications, the tissue to be targeted for homogenization is deep within intervening tissues and the acoustic path may be obstructed by bones or surgical instruments such as imaging probes that totally block acoustic energy transmission. When this occurs, the pressures necessary to initiate and sustain an energetic bubble cloud are not sufficient. Bone aberration of Histotripsy acoustic pulses can be compensated for by increasing the pulse intensity according to the teachings of US Patent App. No. 13/570,708, titled "Lesion Generation Through Bone Using Histotripsy Therapy Without Aberration Correction". Regardless, blockage of energy by bone or instrument blockage reduces the efficiency of therapy transducers and increasing the energy level to compensate can cause unwanted tissue heating at the target focal point and in intervening tissues between the skin surface and the target tissues.

The solutions described below also apply to the design and design methods of therapy transducers of high intensity focuses ultrasound (HIFU) systems. In HIFU, heating tissues in the target focal point is the goal; however, bone blockage is a much bigger problem because the duty cycle and hence the amount of energy delivered is much higher than Histotripsy. Blocking of HIFU acoustic pulses at the bone - soft tissue interface can cause thermal injury to the bone and adjacent soft tissue.

Histotripsy has been shown to be effective in homogenizing prostate tissue in canine studies conducted by scientists at the University of Michigan (U of M). In those studies, the Histotripsy therapy transducer was focused through the abdomen to target the prostate. A transabdominal approach will not work for humans because the prostate is beneath the pubic bone such that acoustic energy from the transducer would be totally blocked. A larger opening for non-invasive Histotripsy treatment of the prostate is provided through the perineal window. The perineal window provides a larger aperture to treat through, but the sound energy falls off with distance from the target which is significantly further from the transducer in a transperineal approach compared with a transabdominal approach in canines. The perineal window is framed by bones at the base of the pelvis such as the coccyx posteriorly, the pubic arch anteriorly, and the ilium laterally.

Furthermore, Histotripsy is guided by ultrasound imaging by placing the imaging probe in the rectum which may interfere with acoustic transmission, physically limits the potential size (diameter) of the transducer and forces the position of the therapy transducer anteriorly causing even more significant acoustic blockage by the anterior pelvic bones. The pelvic bones and imaging probe block sound waves so they are the limiting factor in defining the available window for sound transfer. In order for enough sound energy to pass through this aperture to produce cavitation in the prostate, the transducer will need to be optimized in size, shape, and orientation. To this end, we have developed design methods and designed custom transducers that are described in detail below. The invention is defined in the claims. Other embodiments are merely exemplary.

### SUMMARY

According to the present invention there is provided an ultrasound therapy system for treatment of a prostate through a perineal window of a patient, comprising;
an ultrasound therapy transducer sized and configured to be positioned adjacent to a perineum of the patient and generate and focus ultrasound pulses on the prostate, the ultrasound therapy transducer including an opening disposed along or near a perimeter of the ultrasound therapy transducer; and
a transrectal imaging transducer disposed in the opening of the ultrasound therapy transducer, wherein the opening of the ultrasound therapy transducer allows for linear, pan, and tilt movements of the ultrasound therapy transducer with respect to the transrectal imaging transducer without contact or interference from the transrectal imaging transducer.

In some embodiments, the therapy transducer has a diameter ranging from 80mm to 180mm, a focal distance ranging from 70mm to 150mm, and an F-number below 1.2.

In one embodiment, the opening of the ultrasound therapy transducer has a shape selected from the group consisting of round, semi-circular, square, rectangular, triangular and flat.

In another embodiment, the opening of the ultrasound therapy transducer is configured to accommodate movement of the ultrasound therapy transducer to position a therapy focus of the ultrasound pulses in all regions of the prostate without interfering with the transrectal imaging transducer.

In another embodiment, the ultrasound therapy transducer comprises a "heart shaped" transducer, the ultrasound therapy transducer having a wide end adapted to be placed on a posterior portion of the perineum and a narrow end adapted to be placed on an anterior portion of the perineum, the opening of the ultrasound therapy transducer being disposed on the wide end of the ultrasound therapy transducer.

In one embodiment, the ultrasound therapy transducer comprises a single ultrasound transducer element.

In another embodiment, the ultrasound therapy transducer comprises a plurality of ultrasound transducer elements. In some embodiments, the plurality of ultrasound transducer elements are round. In another embodiment, the plurality of ultrasound transducer elements are hexagonal.

In one embodiment, the transrectal imaging transducer comprises a distal section and an intermediate section proximate to the distal section, the distal section having a first diameter larger than a second diameter of the intermediate section. In another embodiment, the transrectal imaging transducer further comprises a proximal section proximate to the intermediate section, the proximal section having a third diameter larger than the second diameter of the intermediate section.

In some embodiments, the first diameter is less than or equal to 20mm. In another embodiment, the second diameter is less than or equal to 12mm. In an alternative embodiment, the first diameter ranges from 15mm to 20mm and the second diameter ranges from 6mm to 12mm. In one embodiment, the first and third diameters range from 15mm to 20mm and the second diameter ranges from 6mm to 12mm.

In some embodiments, the ultrasound therapy transducer is configured to deliver Histotripsy therapy pulses that form cavitational microbubbles within the prostate at a focal zone of the ultrasound therapy transducer to mechanically fragment prostate tissue without damaging surface tissue or intervening tissue.

A method of treating a prostate through a perineal window of a patient with Histotripsy therapy is also provided, comprising positioning an ultrasound therapy transducer adjacent to a perineum of the patient, positioning a transrectal imaging probe in a rectum of the patient, the transrectal imaging probe being received by an opening in the ultrasound therapy transducer, adjusting a linear, pan, or tilt position of the ultrasound therapy transducer relative to the imaging probe without interference from the imaging probe, obtaining an ultrasound image of the prostate with the imaging probe, and generating Histotripsy pulses that are minimally blocked by bones of the perineal window when focused on the prostate to treat the prostate.

In some embodiments, generating Histotripsy pulses comprises generating short (< 20 µsec), high pressure (peak negative pressure > 10 MPa) shockwave ultrasound pulses at a duty cycle < 5%.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
Fig. 1 is a CT scan of a therapy transducer pointed at a prostate and an imaging probe positioned in a rectum of the patient.
Fig. 2 is a schematic drawing of a therapy transducer.
Fig. 3 is a 3D projection of a transducer shape projected outwards from the prostate through the perineal window.
Fig. 4 is one embodiment of a transducer design.
Fig. 5 is another transducer design having a cutout for an imaging probe.
Figs. 6A-6C illustrate various configurations of a therapy transducer having multiple elements.
Fig. 7 is another embodiment of a therapy transducer.
Fig. 8 illustrates one embodiment of a therapy transducer having a cutout for an imaging probe.
Fig. 9 shows another embodiment of a therapy transducer.
Figs. 10A-10C show another embodiment of a therapy transducer.
Figs. 11A-11B illustrate 4 targets within a prostate at three different depths.
Fig. 12 is a table that summarizes acoustic transmission blockage from twelve positions within a prostate model.
Fig. 13 shows another embodiment of a therapy transducer.
Fig. 14 illustrates two embodiments of an imaging probe.
Fig. 15 shows three embodiments of a therapy transducer.
Fig. 16 shows the percentage blockage results for seven individual points for various specimens.
Figs. 17A-17G illustrate how the therapy transducer can move relative to the imaging transducer with a movement mechanism.

### DETAILED DESCRIPTION

Histotripsy therapy is particularly well suited for treatment of prostate tissue in a male patient. Treatment of the prostate with Histotripsy can be improved by optimizing the Histotripsy transducer design for the specific anatomy of the prostate including the perineal window. In order to design an optimal therapy transducer for non-invasive treatment of the prostate through the perineal window, the pelvic bones (coccyx, pubic arch, ilium and sacrum), with the trans-rectal ultrasound imaging probe in place, were modeled from a series of cadaver CT scans. The perineal window was then back projected from the prostate outward to the therapy transducer position. This method was employed to design an optimally shaped transducer that can generate Histotripsy pulses that are minimally blocked when focused on all aspects of the prostate.

An important lesson learned from back projection modeling was interference from the trans-rectal imaging probe, which pushed the transducer anteriorly, resulting in more severe acoustic blocking from the anterior pelvic bones. Other design considerations were the therapy transducer F-number, which is defined as the focal length divided by the aperture (effective diameter). The focal length should be long enough to treat the entire prostate and the aperture should be large enough for efficient energy delivery to the focal point. It is important to note that pre-focal tissue heating potential is exponentially proportional to the F-number. Consequently a primary design objective is to design a therapy transducer with the lowest possible F-number keeping in mind that acoustic blockage by bones and the imaging probe effectively reduces the F-number.

Various experiments were performed to determine an optimal prostate Histotripsy therapy transducer. CT scans of 5 male cadaver specimens with an ultrasound therapy transducer 100 and transrectal imaging probe 102 were obtained. The specific goals of the study were to determine the effect of the imaging probe placement, average prostate volume, average and minimal perineal window geometry, therapy transducer to apical prostate distance and therapy transducer to bladder neck distance.

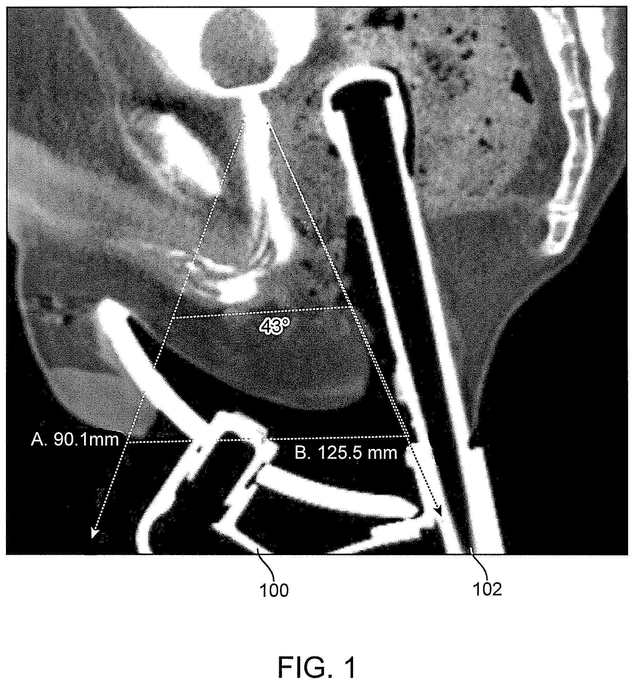

This disclosure describes the use of Histotripsy therapy to generate lesions through rib or bone aberrators without applying any correction mechanisms other than transducer power modulation to compensate for attenuation effects. The ultrasound therapy transducer 100 of Fig. 1 can be configured to generate Histotripsy pulses to deliver Histotripsy therapy to tissue. Histotripsy uses controlled cavitation bubble clouds to induce mechanical tissue fractionation. Histotripsy bubble clouds can be produced by delivering Histotripsy energy to tissue with a Histotripsy transducer, defined by using short (< 20 µsec), high pressure (peak negative pressure > 10 MPa) shockwave ultrasound pulses at a low duty cycle, typically < 5%, minimizing thermal effects. Based on the high echogenicity of cavitating bubble clouds, treatment can also be readily monitored in real time using any conventional ultrasound imaging system, allowing the operator to acknowledge whether cavitation bubble clouds have been generated.

The tissue fractionation effect from Histotripsy therapy occurs when the focal pressure exceeds a certain threshold level at which a cavitation bubble cloud is initiated. Based on this threshold mechanism, Histotripsy therapy can be controlled to generate precise lesions through the ribs or bone provided that the pressure main beam maintains its shape and is above the bubble cloud initiation threshold while secondary lobes resulting from the bone aerator remain below the threshold and thus do not initiate a cavitation bubble cloud.

Still referring to Fig. 1, an ultrasound therapy transducer 100 and transrectal imaging probe 102 were positioned at the perineum and inserted in the rectum, respectively, and then placed in the CT scanner. CT scan data from the 5 cadaver specimens provided distances and angles from positions in the prostate volume past the pelvic bones and perineal window to positions on the therapy transducer. Angle and distance measurements were made to determine accessibility from the transducer to the prostate bladder neck (the farthest target from the transducer surface). The optimal outer diameter (OD) and focal length for a therapy transducer were determined for each specimen. The F-number = OD/Focal length was calculated for each specimen and the average determined, as shown in Table 1.

**Table 1 - Cadaver specimen data (in mm) with average dimensions and F-number:**

| **Cadaver Specimen** | **OD** | **Focal Length** | **F-number** |
|---|---|---|---|
| **1** | 90.1 | 125.5 | 1.393 |
| **2** | 124.2 | 136.1 | 1.096 |
| **3** | 94.2 | 101.7 | 1.080 |
| **4** | 102.4 | 117.0 | 1.143 |
| **5** | 103.7 | 119.4 | 1.151 |
| **Average** | 102.9 | 119.9 | 1.172 |

Based on the data from Table 1, in one embodiment, an optimal outer diameter (OD) for a prostate histotripsy ultrasound therapy transducer can be 105 mm having a focal distance of 120mm for a round therapy transducer configured and optimized for prostate therapy through the perineal window. According to the results from this experiment, in some embodiments, the ultrasound therapy transducer can have an outer diameter ranging from approximately 90mm to 125mm, with a focal length ranging from 100mm to 140mm and an F-number ranging from 1.00 to 1.4. IN another embodiment, the therapy transducer can have an outer diameter ranging from approximately 75mm to 150mm, a focal length ranging from 80mm to 160mm, and an F-number ranging from 0.5 to 1.5.

The workable distance for a histotripsy therapy transducer is the distance from the focal point of the transducer to the closest point on the transducer surface, if the surface is flat. A workable distance LW is calculated using Pythagorean Theorem calculation where:
LW = Workable Distance
Lf = Focal Distance
LOD = Overall Diameter
LW = √Lf²-(LOD/2)²

Using the focal distance and OD described above, the LW = √120²-(105/2)²= √14400-2756.25=√11643.75=107.90mm

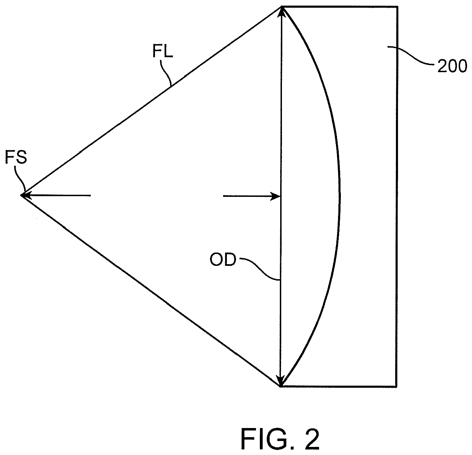

Fig. 2 illustrates a schematic of a sample transducer 200 having a focal length FL, a focal spot FS, a working distance WD, and an OD, illustrating the parameters described above.

Based on these measurements and calculation, the F-number of the optimal transducer, defined as the focal length divided by the diameter of the transducer, equals 1.14. Ideally, the F-number can be ≤ 1.0 to avoid pre-focal heating and unintended tissue damage. It was determined that the position of the rectal imaging probe interferes with the optimal position of the round therapy transducer. Applicants have determined through experimentation that higher F-numbers substantially increase the risk of pre-focal heating and tissue damage. The therapy transducers of the present disclosure have been designed so as to have a sufficiently low F-number and an effective pulse sequence that homogenizes tissue efficiently without pre-focal heating.

In another experiment, a second series of two caver specimens were imaged with a shorter mock imaging probe that would result in less interference with the therapy transducer position. In the first experiments presented above, the imaging probe was 30 cm in length. In the second series, imaging probes of 15 cm and 20 cm were evaluated. The F-number = OD/Focal length was calculated for each specimen and the average determined as shown in Table 2:

**Table 2**

| **Cadaver Specimen** | **OD** | **Focal Length** | **F-number** |
|---|---|---|---|
| **1** | 190.1 | 97.10 | 0.5101 |
| **2** | 122.0 | 106.0 | 0.869 |
| **Average** | 156.1 | 101.55 | 0.690 |

Based on the data from this data set, the OD can range from 120mm to 190mm (averaging to approximately 155mm) and the focal distance can range from approximately 95mm to 110mm for a round therapy transducer designed for prostate therapy. In this embodiment, the F-number = 0.70 and the workable distance is 78.3 mm for an optimal therapy transducer design based on the measurements from the cadaver specimens in this series using the equations above.

The results from the series of cadaver specimen experiments above are very different. They show that in the first experiment, the imaging probe interfered with optimal positioning of the ultrasound therapy transducer forcing its position up (anteriorly), thereby resulting in interference with the therapy transducer from the pelvic bones and increasing the F-number of the therapy transducer. The experiments indicate that minimizing interference of the imaging transducer with the therapy transducer can help to lower the F-number of the therapy transducer.

In additional experiments, data from the cadaver specimen experiments was modeled in 3D to determine an optimal shape of a Histotripsy therapy transducer particularly well suited for treatment of the prostate.

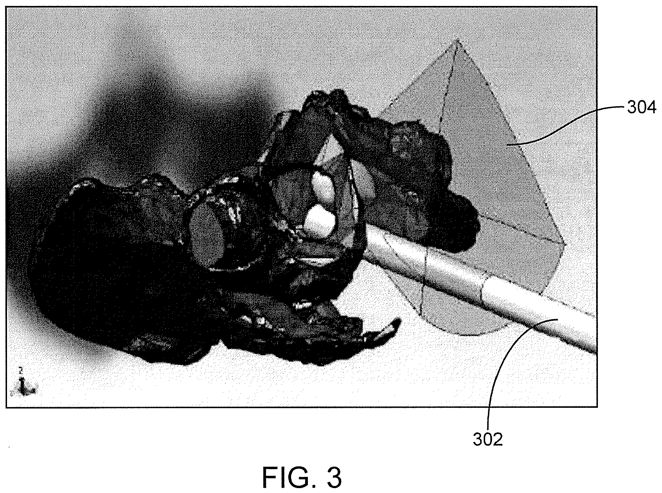

DICOM files from the cadaver studies were transferred into SolidWorks (mechanical engineering software tool for 3D modeling). The shape of the ideal transducer was determined from back projections from the bladder neck point on the prostate (point farthest from the therapy transducer) through the boney window of the pelvis. Models of the prostate and imaging probe were positioned precisely inside the reconstructed pelvis. Projections from the bladder neck base of the prostate through the pelvic bones and around the imaging probe were made on the 3D models. Fig. 3 illustrates a 3D projection 304 as it is projected outwards from the prostate through the perineal window. The interaction of the rectal imaging probe 302 is also shown with respect to the 3D projection.

Based on the 3D modeling, one potentially advantageous shape for a therapy transducer based on these projections is somewhat triangular. The transducer could be placed over the trans-rectal imaging probe or a larger triangular transducer that has an opening or cutout near the bottom to accommodate the imaging probe. Based on the 3D modeling, in one embodiment measurements from the tip of the triangle to the surface of the rectal imaging probe were obtained for 10cm, 11cm, 12cm, and 13cm, from the bladder neck, as shown in Table 3.

**Table 3**

| **Prostate to transducer distance** | **Imaging probe to top distance** |
|---|---|
| 13cm | 14.83 |
| 12cm | 14.05 |
| 11cm | 12.89 |
| 10cm | 11.9 |
| **Average** | **13.42** |

A second 3D model was constructed in SolidWorks CAD software using DICOM files from the cadaver CT scans. The shape of another transducer based on back projections from the bladder neck point on the prostate through the reconstructed pelvic window was determined. Trans-rectal imaging probe and prostate models were inserted in the reconstructed anatomy. Based on projections through the perineal window and the ideal shape of the opening, one embodiment of a transducer comprises a complex triangle with a bottom opening or cutout for placement of the trans-rectal imaging probe.

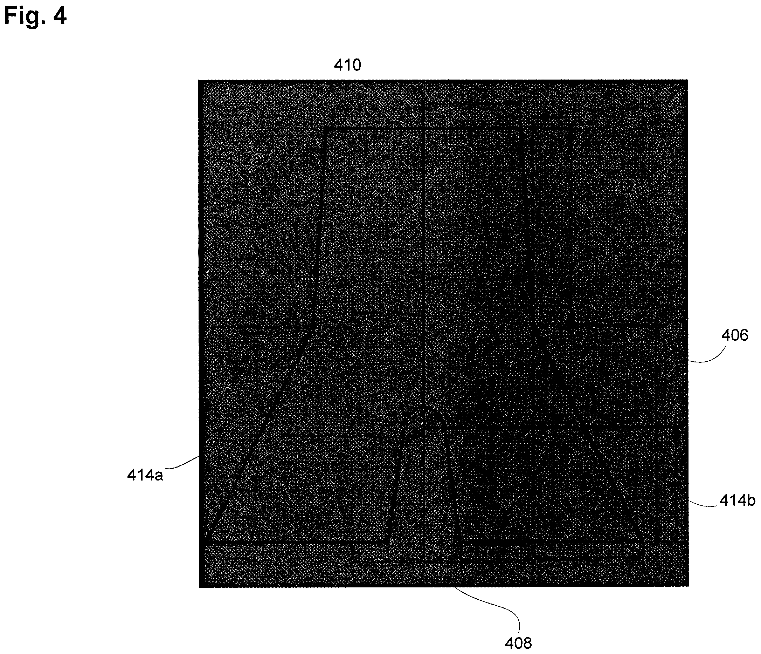

One example of this complex triangle shaped ultrasound transducer is shown in Fig. 4. As shown, the transducer 406 can include a cutout 408 sized and configured to receive an imaging transducer, such as a rectal imaging probe. The cutout 408 can be positioned along or near a perimeter of the transducer. In the embodiment shown, the cutout is positioned along a wide end of the transducer. The transducer can also include a flat top surface positioned along a narrow end 410 of the transducer, attached to a pair of slanted surfaces 412a and 412b. Approximately midway down the transducer, the slanted surfaces can join second slanted surfaces 414a and 414b, angled further outwards than the slanted surfaces. The transducer can further include a flat bottom surface that includes opening or cutout 408 for the imaging probe.

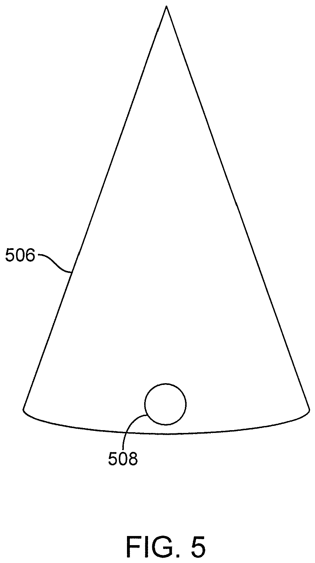

Fig. 5 illustrates another potential transducer shape based on 3D modeling that would be suited for transmitting histotripsy therapy through the perineal window into the prostate. As shown in Fig. 5, the transducer 506 can comprise a relatively triangular shape having a cutout 408 towards the bottom of the transducer to accommodate a transrectal imaging probe. The triangular shape is shown with a curved bottom surface, but it should be understood that any shaped bottom surface (e.g., flat, etc.) can be used. In the example of Fig. 5, the cutout 508 comprises a circular shape disposed near the perimeter of the transducer.

Fabrication of a therapy transducers having complex shapes described above should take practical and economic factors into consideration. The cheapest and most common ultrasound therapy transducers on the market today are simple round shapes. They are typically fabricated from piezo-electric materials that are shaped to provide a specified aperture and focal distance. The piezo-electric component often is cut into smaller elements to create a phased array transducer in which each element is pulsed with electrical signals from separate amplifier drive systems, which enables electronic steering of the transducer. In order to generate the large voltages desired for Histotripsy transducers, each piezo-electric transducer element of a Histotripsy therapy system must have a sufficiently high impedance to match with a lower impedance switching amplifier through an oscillating LC matching network. High impedance transducer elements typically have a small surface area, and large transducer surfaces required for non-invasive Histotripsy applications require large apertures (diameter) and therefore, a relatively large number of independently driven elements.

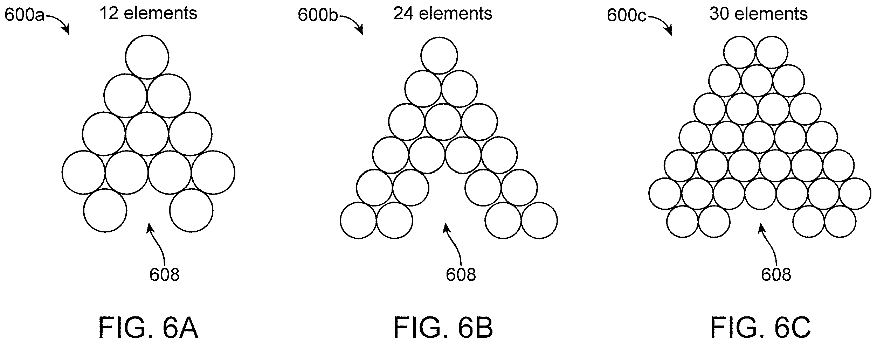

One method of fabricating a therapy transducer with multiple elements is to use a plurality of round discs. This fabrication method can be used to create designs that more closely approximate the unique shapes from cadaver specimen projections described above. Figs. 6A-6C show three transducer designs 600a, 600b, and 600c, respectively, fabricated from discs to approximate the unique triangular shape with an imaging probe cutout or opening. The discs can be separated with minimal spacing (e.g., 1mm spaces between discs). Transducer 600a can comprise 12 elements, transducer 600b can comprise 24 elements, and transducer 600c can comprise 30 elements. Each of the transducers can form a somewhat triangular or "heart-shaped" design and can further include an opening or cutout 608 positioned near the perimeter of the wide end of the transducer. As described herein, a "heart-shaped" transducer can generally have a narrow curved end and a wide end opposite the narrow end with two curved portions on either side of a cutout or opening. Table 4 describes the total number of elements of various sizes required to construct a therapy transducer of optimal dimensions based on the modeling described above.

**Table 4**

| Total number of elements | Disc Diameter | Overall OD | Total Surface Area | Element Surface Area | Efficiency |
|---|---|---|---|---|---|
| 12 | 32.63 | 133.78 | 16,013.68 | 10,308.18 | 74% |
| 24 | 23.07 | 140.73 | 15,510.80 | 10,492.12 | 68% |
| 30 | 20.64 | 144.48 | 16,351.48 | 10,777.99 | 66% |

The surface area efficiency of round elements is approximately 66% - 74%. The surface area efficiency may be improved using multiple hexagonal elements that accommodate positioning of the rectal imaging probe and minimize pelvic bone interference. A 12 hexagonal element configuration where each element has an 836.2mm² surface area with an outer length (OL) of 33.39mm has 94% coverage efficiency when 1mm spaces separate the elements, as described in Table 5.

**Table 5**

| Total number of elements | Disc diameter Hexagon OL | Overall OD | Total Surface Area | Element Surface Area | Efficiency |
|---|---|---|---|---|---|
| 12 round | 32.63 | 133.78 | 16,013.68 | 10,308.18 | 74% |
| 12 Hexagon | 33.39 | 137.56 | 10,641.36 | 10,034.16 | 94% |

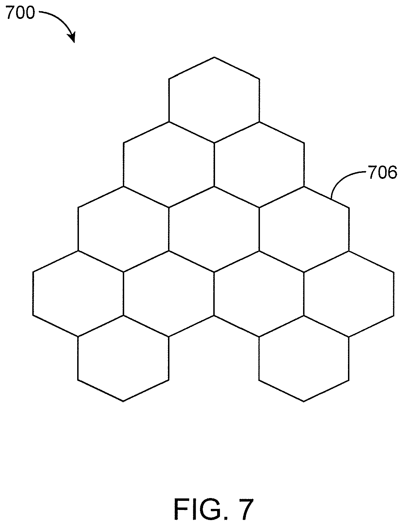

Fig. 7 illustrates a similarly shaped transducer 700 being fabricated from multiple hexagonal shaped elements 706 to increase efficiency of the transducer design. In one particular embodiment, the transducer can have a height of approximately 137mm and a width of approximately 118mm at the widest point. Round piezo-electric elements are typically more readily available than hexagonal elements; consequently a transducer fabricated from round elements is more practical and inexpensive than the hexagonal formed transducer of Fig. 7.

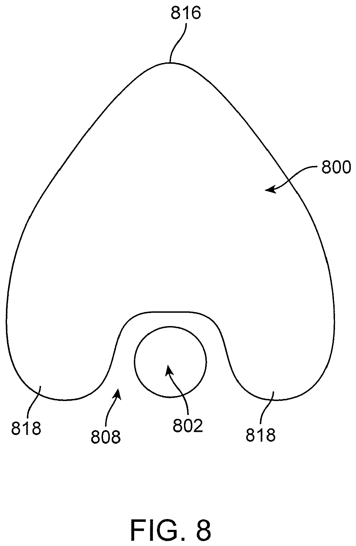

Fig. 8 illustrates yet another embodiment of an ultrasound therapy transducer 800 having a "heart-shaped" design with a cutout 808 to receive an imaging transducer 802, such as a rectal imaging probe. The transducer of Fig. 8 is substantially triangular in shape, but has a curved top portion 816 and curved lower portions 818 near the opening 808, so as to accommodate the use of round transducer elements during manufacturing.

A theoretical analysis was performed to assess the percentage of beam blockage for the shaped transducers shown above in Figs. 4-8 compared to the beam blockage for a circular transducer (disk) from 6 positions within the prostate. The percentage of blockage for the circular transducer vs. custom shaped transducers (Figs. 4-8) is shown in Tables 6 and 7.

**Table 6**

| position | Circular transducer Three axis linear motion | Circular transducer Pan/tilt plus one axis linear motion | Circular transducer Two goniometers plus one axis linear motion |
|---|---|---|---|
| 1 | 0% | 0% | 0% |
| 2 | 0% | 0% | 0% |
| 3 | 0% | 0% | 0% |
| 4 | 40% | 20% | 10% |
| 5 | 80% | 100% | 60% |
| 6 | 60% | 30% | 25% |

**Table 7**

| position | Heart-shaped transducer Three axis linear motion | Heart-shaped transducer Pan/tilt plus one axis linear motion | Heart-shaped transducer Two goniometers plus one axis linear motion | Heart-shaped transducer One Angled goniometer plus two axis linear motion |
|---|---|---|---|---|
| 1 | 0% | 0% | 0% | 0% |
| 2 | 0% | 0% | 0% | 0% |
| 3 | 0% | 0% | 0% | 0% |
| 4 | 33% | 20% | 8% | 2% |
| 5 | 65% | 100% | 32% | 26% |
| 6 | 38% | 18% | 11% | 10% |

Blockage of the acoustic signal was made for each transducer using different combinations of transducer positioning stages (linear, pan/tilt, and goniometers). In the positions 1, 2, and 3, which are closest to the skin surface there is no blockage for either transducer or positioning method. For the deeper positions 4, 5, and 6 the blockage is significant.

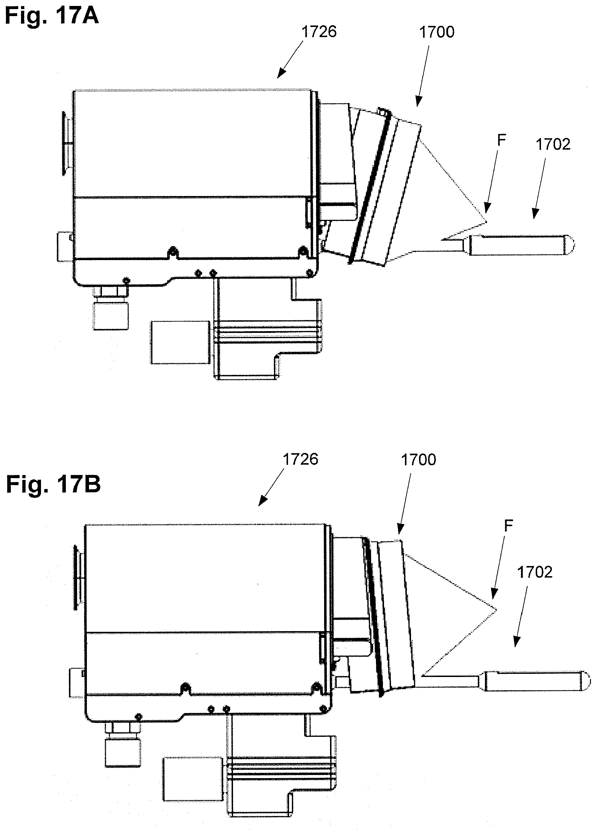

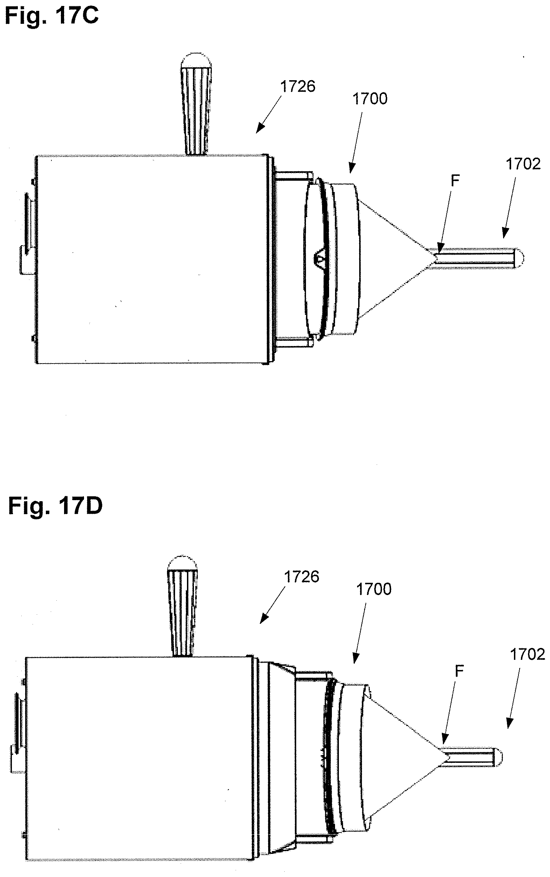

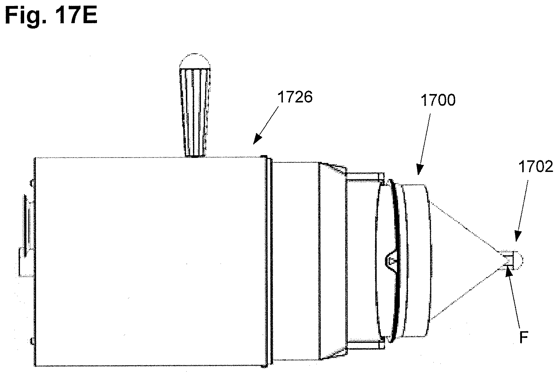

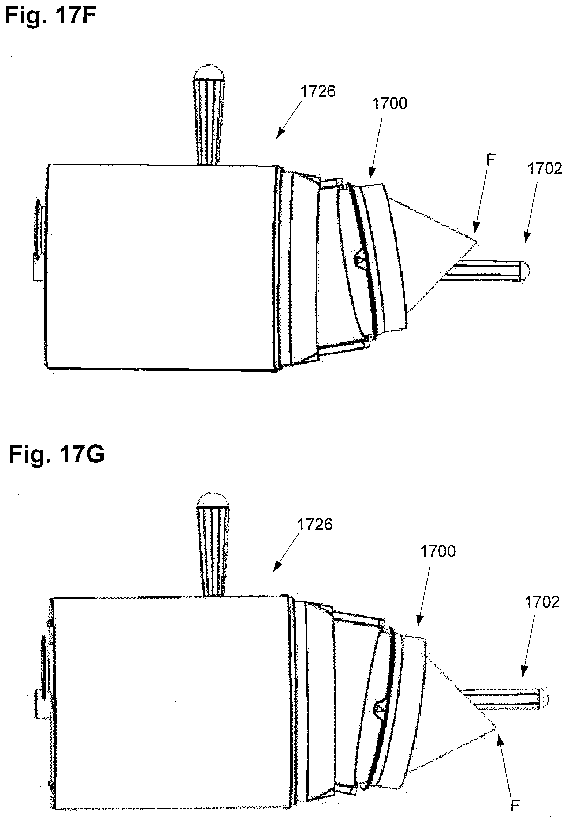

Figs. 17A-17G illustrate one embodiment of an ultrasound imaging system comprising an ultrasound therapy transducer 1700 and an ultrasound imaging transducer 1702. Figs. 17A-17F also show movement mechanism 1726, which can be configured to control linear, pan, and tilt movements of the therapy transducer with respect to the imaging probe. These movements adjust a position of the focal point F of the therapy transducer with respect to the imaging probe. In Figs. 17A and 17B (side view), the therapy transducer 1700 is shown tilting down and up, respectively, with respect to the imaging transducer 1702. In Figs. 17C, 17D and 17E (top down view), the therapy transducer 1700 is shown in the linear back, middle range, and forward positions, respectively, with respect to the imaging transducer 1702. In Figs. 17F and 17G (top down view), the therapy transducer 1700 is shown panning left and right, respectively, with respect to the imaging transducer 1702. The ultrasound imaging transducer 1700 can include any of the embodiments described herein wherein the transducer includes a notch or opening disposed near a perimeter of the transducer to accommodate and receive the imaging transducer.

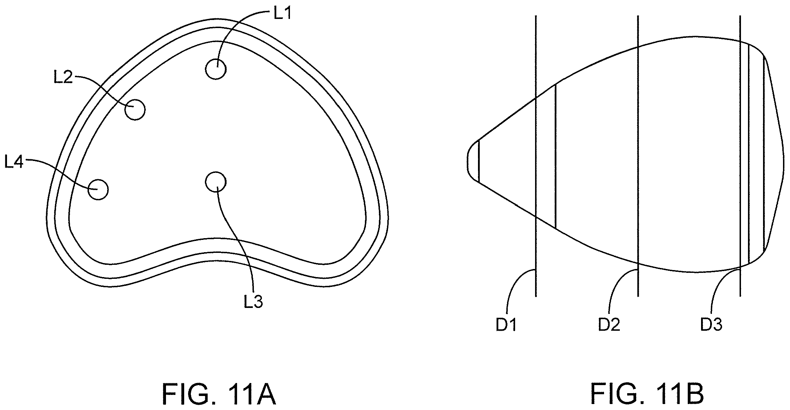

Referring to Tables 6 and 7 above, position 1 can be defined as treating the front of the prostate (the front of the prostate can be shown by depth D1 in Fig. 11B). This can be achieved by translating the therapy transducer back (as in Fig. 17C) with tilt and pan in the approximate "zero" position. Position 2 can be defined as treating the middle-center of the prostate. This can be achieved by translating the therapy transducer into the middle range (as in Fig. 17D) with tilt and pan in the approximate "zero" position. Position 3 can be defined as treating the middle-bottom of the prostate. This can be achieved by translating the therapy transducer into the "zero" position with the therapy transducer tilted down (as in Fig. 17A) and pan in the approximate "zero" position. Position 4 can be defined as treating the middle-top of the prostate. This can be achieved by translating the therapy transducer into the "zero" position with the therapy transducer tilted up (as in Fig. 17B) and pan in the approximate "zero" position. Position 5 can be defined as treating the middle-left/right of the prostate. This can be achieved by panning left and right (as in Figs. 17F-17G). Position 6 can be defined as treating the back of the prostate. This can be achieved by translating the therapy transducer forward (as in Fig. 17E) with tilt and pan in the approximate "zero" position.

The movement mechanism 1726 can be an automated or manually controlled system for controlling the linear and pan/tilt movement of the therapy transducer with respect to the imaging probe. The mechanism can include, for example, gears, motors, hardware, and software configured to advance and retract the therapy transducer linearly, and steer the direction of the transducer up/down and left/right. In many embodiments, the cutout or notch of the therapy transducer allows for movement of the therapy transducer without interference from the imaging probe or touching the imaging probe. During treatment, the therapy transducer can be controlled through each of the 6 positions and Histotripsy therapy can be applied to each position to treat the entire prostate.

The best results occur when two goniometers are employed to position the transducer. The results are better with the heart-shaped transducer primarily for positions 4, 5, and 6. Also note that the best results with the heart-shaped transducer occur when one angled goniometer and two linear stages are employed. This results from offsetting the axis of rotation of the goniometer by a few centimeters below (posterior) the normal position. This observation further illustrates the importance of the cut out around the imaging probe. For this configuration the blockage is minimal.

Table 8 shows the percentage of improvement that the custom shaped (triangular, heart shaped, etc.) transducer delivered compared to the circular transducer for each of the 6 positions in the prostate.

**Table 8**

| | 3 Axis Lin | Pan/Tilt + 1 Axis Lin | 2 Gon + 1 Axis Lin |
|---|---|---|---|
| 1 | 0% | 0% | 0% |
| 2 | 0% | 0% | 0% |
| 3 | 0% | 0% | 0% |
| 4 | 17% | 0% | 20% |
| 5 | 19% | 0% | 47% |
| 6 | 37% | 40% | 56% |

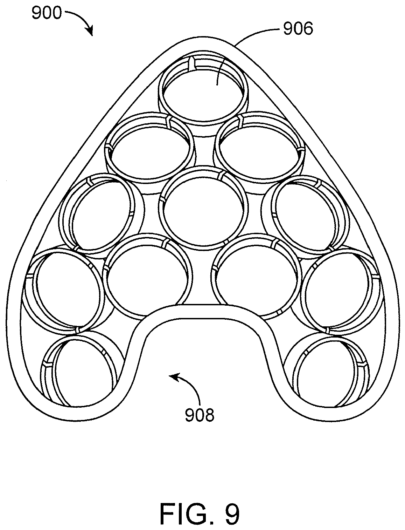

The custom heart-shaped transducer of Fig. 8 can be fabricated with multiple round transducer elements, as shown in Fig. 9 as ultrasound therapy transducer 900 comprising round transducer elements 906. As shown, the round transducer elements can be arranged to form the heart-shaped transducer having an opening or cutout 908 on the wide end of the transducer. Although the elements in Fig. 9 are shown as being round, in some embodiments, the transducer elements can also be hexagonal. In order to reduce the cost of the transducer and rapidly build custom transducers, the transducers can be built using multiple small, shaped elements. In some embodiments, a housing of the ultrasound therapy transducer can support an arrangement of 3 cm diameter circular discs with a minimum of separation between the transducers (e.g., approximately 1mm or less). The discs can either be concave to focus the sound energy at the target or they can be flat in order to achieve the same effect. Flat discs have the advantage of being much less expensive than concave focusing discs. When flat discs are used, a lens comprising of a molded plastic material can be placed over the transducer element.

In order to optimize the F-number (F-number = Focal Length/Aperture (diameter)) and focal length (required to reach deep prostate target areas) the aperture or diameter of the transducer must be maximized. A circular transducer provides a larger aperture than the custom designs described above. In order to optimally position a circular transducer, a cut out was made at the bottom of the circular transducer to accommodate the rectal imaging probe and allow a more posterior position of the transducer to minimize acoustic transmission blocking by the anterior pelvic bones. Although bone blockage will effectively reduce the aperture of the round transducer, its efficiency is maximized for any given target within the prostate and Histotripsy can accommodate for aberration from bone blockage.

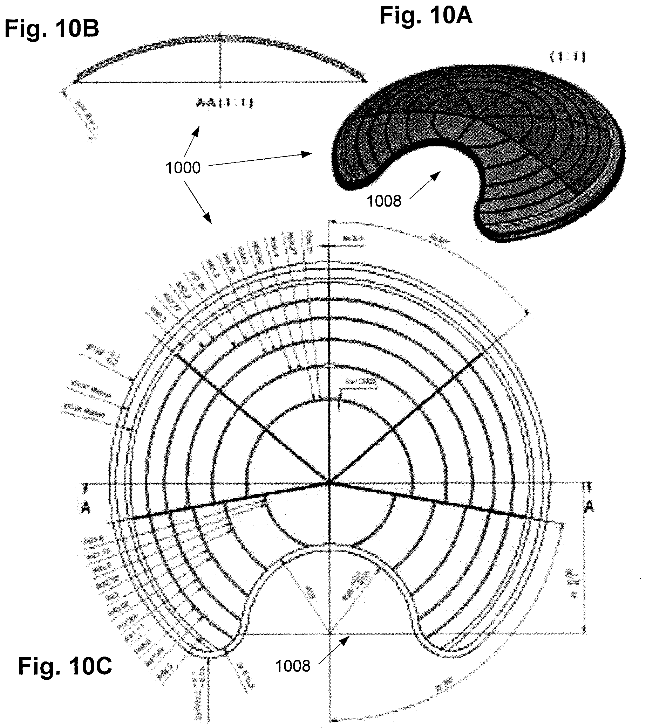

In another embodiment, a circular ultrasound therapy transducer 1000 was designed with a cut-out 1008 for the rectal imaging probe on the bottom, as shown in Figs. 10A-10C. Fig. 10A shows a three-quarter view of the transducer, Fig. 10B shows a side view of the transducer, and Fig. 10C shows a schematic drawing of the transducer. In on particular embodiment, the transducer can have a diameter of approximately 140mm and an active transducer surface diameter of 125mm. The focal length can be approximately 130mm and the workable distance approximately 11 cm. The transducer can be fabricated from a single piezo electric element that is cut and divided into separate elements of equal surface area and input impedance. In the illustrated embodiment, the transducer was designed with 36 transducer elements in order to substantially increase the input impedance of each element and enable development of sufficient voltage input to each element. The F-number based on the active area OD of the aperture width of 125mm and focal length of 130mm is 1.04. The F-number across the cutout, or in the vertical dimension, is 1.69.

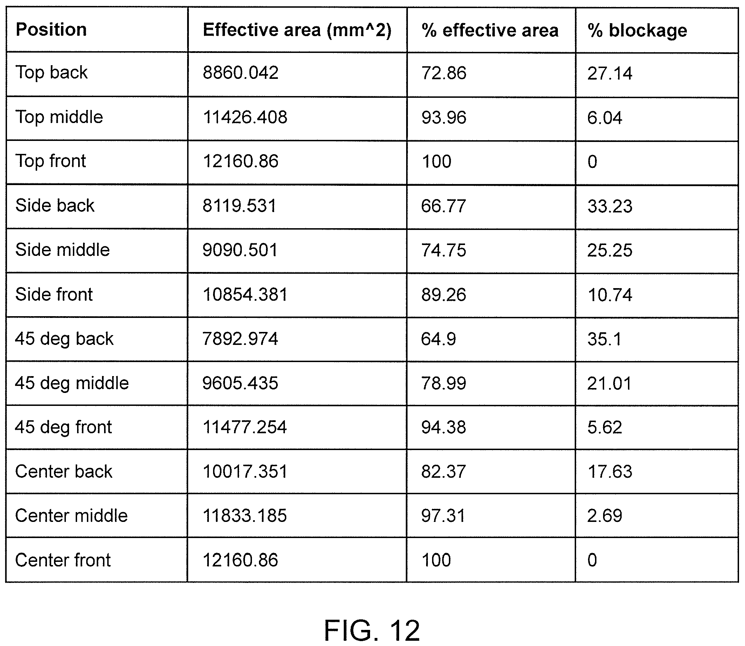

The percent blockage of acoustic output by bone was determined for treatment of different areas of the prostate using computer modeling from cadaver specimens from the experiments presented above. SolidWorks was used to model the pelvis bones, tailbone, imaging probe, transducer, focal cone of the transducer and prostate. The prostate shape was based on anatomic models. The pelvic bone, rectal imaging probe and prostate were placed in their anatomic positions and the transducer was maneuvered to focus on the points of the prostate defined below. Blockage of acoustic transmission by bone was modeled by back projecting the transducer focus onto the plane defined by the front edge of the transducer. Any projection that is blocked by bone is left off the projection. The percent effective area was calculated by dividing the effective area by the area of the active face of the transducer. Percent blockage with the transducer of Figs. 10A-10C was determined at four (4) locations L1, L2, L3, and L4 in each of three (3) depths D1 (front), D2 (middle), and D3 (back) in the prostate, as shown in Figs. 11A and 11B. Fig. 12 is a table that summarizes acoustic transmission blockage from twelve positions within the prostate model. As expected, the targeted locations farthest from the transducer projected up to 35.1% blockage in the circular transducer model.

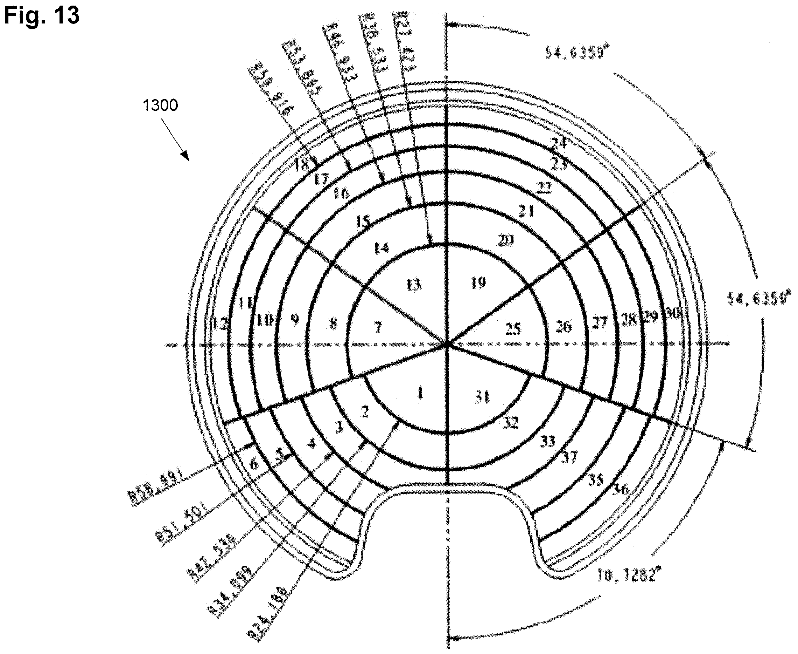

In another embodiment, the circular transducer of Fig. 10 was modified by reducing the focal length to 110mm, increasing the aperture to 130mm and decreasing the cut-out by about 15mm. This new transducer design is shown as ultrasound therapy transducer 1300 in Fig. 13. Once again, the ultrasound therapy transducer can comprise a plurality of individual transducer elements, in this example having 36 transducer elements (individually numbered on Fig. 13). Table 9 compares the two custom circular transducers of Figs. 10 and 13. These changes decreased the F-number from 1.04 to 0.85 at the main diameter and from 1.69 to 1.12 at the cut out. The modified transducer of Fig. 13 also operated optimally and was driven at 750 KHz. The lower frequency pulses have lower attenuation in tissue than higher frequency pulses. The modified custom therapy transducer of Fig. 13 has 36 elements of equal size that are driven by individual amplifier/matching network circuits.

**Table 9**

| | Freq | OD | Active OD | F-Number | Cut-Out | Active OD | F-Number |
|---|---|---|---|---|---|---|---|
| Original (Fig. 10) | 1MHz | 140 | 125 | 1.04 | 55 | 77 | 1.69 |
| Modified (Fig. 13) | 750KHz | 145 | 130 | 0.85 | 39 | 98 | 1.12 |

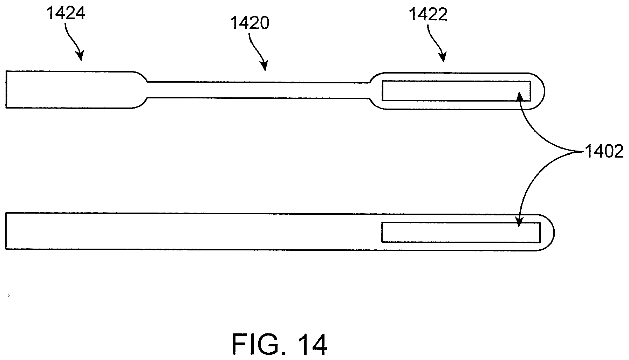

The decreased size of the imaging probe cutout in the transducer design of Fig. 13 necessitated a design modification to the imaging probe. Referring to Fig. 14, two rectal imaging probes 1402 are shown. The top imaging probe includes an intermediate section 1420 of the imaging probe that passes through the cut-out with a reduced diameter relative to the distal section 1422 and proximal section 1424 to accommodate the smaller transducer cutout and allow for pan and tilt motion of the therapy transducer with respect to the imaging transducer. The narrow segment can be positioned in the anus while the thicker imaging transducer section can be positioned in the rectum posterior to the prostate. The lower probe shown in Fig. 14 is a standard imaging probe without the narrow section. In some embodiments, the proximal and distal sections can have a diameter ranging from 15mm to 20mm, and the intermediate section can have a diameter ranging from 6mm to 12mm. This is in comparison to the standard probe shown on the bottom of Fig. 14, which typically has a constant diameter of approximately 18mm.

The circular transducer designs were verified in simulation and excised tissue studies to determine the duty cycle threshold that would not create a thermal lesion. It is important to note that Histotripsy, when delivered effectively, does not thermally injure tissue at the focal point or in the tissue between the skin surface and focal point. A thermal lesion is created when the tissue temperature exceeds 43°C for a prolonged period of time and the transducer is operating with parameters creating a thermal, rather than Histotripsy, effect. Thermal lesions also are created at higher temperatures with shorter exposure. It was determined in tissue studies that the non-thermal duty cycle threshold is much higher for the tested transducer with an F-number between 1.04 and 1.69 than for the tested transducer with a F-number between 0.85 and 1.12. Animal studies confirmed that the lower F-number transducer had a Histotripsy duty cycle threshold that was approximately 6 times higher than the transducer with a higher F-Number.

The modified design was tested in cadaver studies to verify that the acoustic window through the pelvic bones was adequate to treat the prostate. It was also important to consider that blockage of the acoustic signal by pelvic bones needs to be overcome by increasing the amplitude and this increase in acoustic energy can increase the risk of thermal injury. For example, a patient with a small perineal aperture, that blocks a higher percentage of the sound energy, will require more acoustic energy than a patient with a larger perineal aperture that blocks less sound energy.

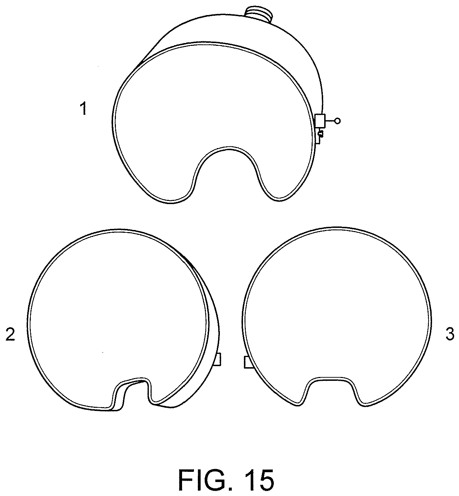

Five cadaver specimens were examined in a manner similar to the methods described above. The imaging probe was placed in the rectum and used to scan the prostate in order to determine its size and location in the pelvis. Four data sets were acquired for each specimen: one data set without the imaging probe and three sets with the three different therapy transducer mockups in place, as shown in Fig. 15. The mockups represent the following transducers which are listed in order of decreasing F-number:
1. Design 1 (140 mm diameter; focal distance = 130 mm)
2. New design 2 (145 mm diameter; focal distance = 125 mm)
3. New design 3 (145 mm diameter; focal distance = 110 mm)

A concern with the shorter focal distance therapy transducers is whether or not they will be able to focus energy at the far end of the prostate next to the bladder neck. Based on cadaver data from this series and past studies, a focal length of 110mm and corresponding working length of 83 mm should enable treatment of the majority of patients. Since this transducer design has the lowest F-number it is considered the best design and it is the subject of the following analysis of acoustic beam blockage during prostate treatment.

DICOM standard/format files were created and saved to disk for all of the specimens. These data sets were then processed and imported into SolidWorks, a 3D software tool. Prostates of each specimen were placed inside the pelvic bone in reference to the imaging probe using CT images as a guide. The following measurements were made:
The dimensions of prostates; length, width and height.

The distance between the furthest back point of the prostate and tail bone (skin)
Orientation of prostate inside the pelvis bone
The distance between the outer surface of the imaging probe and tail bone (skin)
After determining prostate volumes, 7 treatment points were selected in order to perform a geometric analysis to determine the percentage of the acoustic signal emanating from the transducer face is blocked. The 7 treatment points were the back, bottom, middle top, middle right, middle center, middle left, and front of the prostate.

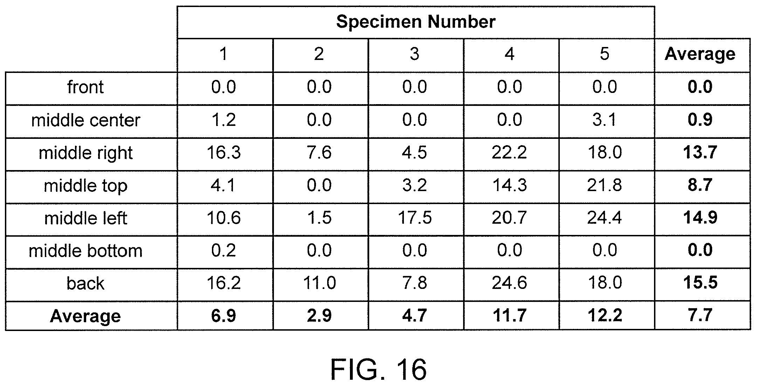

The percentage blockage results for seven individual points for each specimen as shown in the table shown in Fig. 16. Note that the point at the front of the prostate had no blockage for any of the specimens. The same was more or less true for the middle center and middle bottom points. The latter result is a consequence of the fact that the bony blockage is worse towards the upper part of the bony window (the aperture is shaped like an "A") and this point is targeted by angling the transducer downwards. The other points all had between roughly 9% and 15% blockage on average with the worst being the point at the "back" of the prostate (the location of the bladder neck junction). It is also interesting to note from these data that there is a significant difference from subject to subject. Specimen 2 averaged 2.9% blockage (average over the 7 points) while specimen 5 averaged 12.2% blockage. These represented the two extremes from these samples. The overall average for all of the points and all of the specimens was 7.7%. This is well below the 20% blockage value discussed previously, therefore an analysis of the remaining data (images acquired using alternative transducer design mock-ups) was deemed not necessary.

This disclosure represents the first of its kind where human cadaveric subjects have been imaged in the lithotomy position with the goal of determining the size of the bony aperture for BPH or prostate cancer treatment with Histotripsy or any form of therapeutic ultrasound. This study included the Histosonics ultrasound imaging probe and therapy transducer (preferred re-design) positioned as they will be for BPH or prostate cancer treatment in 5 cadaver specimens. The data from this study estimated the amount of blockage that should be expected during prostate treatment for BPH or cancer. It was used to determine the Histotripsy parameters that are safe to use given the expected bony blockages that resulted in reduction of the acoustic transmission aperture and increase in effective F-number.

## Claims

1. An ultrasound therapy system for treatment of a prostate through a perineal window of a patient, comprising;
an ultrasound therapy transducer (800) sized and configured to be positioned adjacent to a perineum of the patient and generate and focus ultrasound pulses on the prostate, the ultrasound therapy transducer including an opening (808) disposed along or near a perimeter of the ultrasound therapy transducer; and
a transrectal imaging transducer (802) disposed in the opening of the ultrasound therapy transducer,
**characterised in that**:
the opening of the ultrasound therapy transducer allows for linear, pan, and tilt movements of the ultrasound therapy transducer with respect to the transrectal imaging transducer without contact or interference from the transrectal imaging transducer.

2. The ultrasound therapy system of claim 1 wherein the therapy transducer has a diameter ranging from 80mm to 180mm, a focal distance ranging from 70mm to 150mm, and an F-number below 1.2.

3. The ultrasound therapy system of claim 1 wherein the opening of the ultrasound therapy transducer has a shape selected from the group consisting of round, semi-circular, square, rectangular, triangular and flat.

4. The ultrasound therapy system of claim 1 wherein the ultrasound therapy transducer comprises a "heart shaped" transducer, the ultrasound therapy transducer having a wide end adapted to be placed on a posterior portion of the perineum and a narrow end adapted to be placed on an anterior portion of the perineum, the opening of the ultrasound therapy transducer being disposed on the wide end of the ultrasound therapy transducer.

5. The ultrasound therapy system of claim 1 wherein the ultrasound therapy transducer comprises a single ultrasound transducer element.

6. The ultrasound therapy system of claim 1 wherein the ultrasound therapy transducer comprises a plurality of ultrasound transducer elements.

7. The ultrasound therapy system of claim 6 wherein the plurality of ultrasound transducer elements are round.

8. The ultrasound therapy system of claim 6 wherein the plurality of ultrasound transducer elements are hexagonal.

9. The ultrasound therapy system of claim 1 wherein the transrectal imaging transducer comprises a distal section and an intermediate section proximate to the distal section, the distal section having a first diameter larger than a second diameter of the intermediate section.

10. The ultrasound therapy system of claim 9 wherein the transrectal imaging transducer further comprises a proximal section proximate to the intermediate section, the proximal section having a third diameter larger than the second diameter of the intermediate section.

11. The ultrasound therapy system of claim 9 wherein the first diameter is less than or equal to 20mm.

12. The ultrasound therapy system of claim 9 wherein the second diameter is less than or equal to 12mm.

13. The ultrasound therapy system of claim 9 wherein the first diameter ranges from 15mm to 20mm and the second diameter ranges from 6mm to 12mm.

14. The ultrasound therapy system of claim 10 wherein the first and third diameters range from 15mm to 20mm and the second diameter ranges from 6mm to 12mm.

## Patentansprüche

1. Ultraschalltherapiesystem zur Behandlung einer Prostata durch ein perineales Fenster eines Patienten, wobei das System Folgendes umfasst:
einen Ultraschalltherapiewandler (800), der so bemessen und konfiguriert ist, dass er neben einem Damm des Patienten angeordnet wird, um Ultraschallimpulse auf der Prostata zu erzeugen und zu fokussieren, wobei der Ultraschalltherapiewandler eine Öffnung (808) aufweist, die entlang oder nahe einem Umfang des Ultraschalltherapiewandlers angeordnet ist; und
einen transrektalen Bildgebungswandler (802), der in der Öffnung des Ultraschalltherapiewandlers angeordnet ist,
**dadurch gekennzeichnet, dass**:
die Öffnung des Ultraschalltherapiewandlers eine lineare, Schwenk- und Kippbewegung des Ultraschalltherapiewandlers in Bezug auf den transrektalen Bildgebungswandler ohne Kontakt mit dem oder Störung durch den transrektalen Abbildungswandler ermöglicht.

2. Ultraschalltherapiesystem nach Anspruch 1, wobei der Therapiewandler einen Durchmesser im Bereich von 80 mm bis 180 mm, eine Brennweite von 70 mm bis 150 mm und eine F-Zahl unter 1,2 aufweist.

3. Ultraschalltherapiesystem nach Anspruch 1, wobei die Öffnung des Ultraschalltherapiewandlers eine Form aufweist, die aus der Gruppe ausgewählt wird, die aus rund, halbkreisförmig, quadratisch, rechteckig, dreieckig und flach besteht.

4. Ultraschalltherapiesystem nach Anspruch 1, wobei der Ultraschalltherapiewandler einen "herzförmigen" Wandler umfasst, wobei der Ultraschalltherapiewandler ein breites Ende aufweist, das dafür ausgelegt ist, auf einem hinteren Teil des Dammes angeordnet zu werden und ein schmales Ende aufweist, das dafür ausgelegt ist, auf einem vorderen Teil des Dammes angeordnet zu werden, wobei die Öffnung des Ultraschalltherapiewandlers am breiten Ende des Ultraschalltherapiewandlers angeordnet ist.

5. Ultraschalltherapiesystem nach Anspruch 1, wobei der Ultraschalltherapiewandler ein einziges Ultraschallwandlerelement umfasst.

6. Ultraschalltherapiesystem nach Anspruch 1, wobei der Ultraschalltherapiewandler eine Vielzahl von Ultraschallwandlerelementen umfasst.

7. Ultraschalltherapiesystem nach Anspruch 6, wobei die Vielzahl von Ultraschallwandlerelementen rund ist.

8. Ultraschalltherapiesystem nach Anspruch 6, wobei die Vielzahl von Ultraschallwandlerelementen sechseckig ist.

9. Ultraschalltherapiesystem nach Anspruch 1, wobei der transrektale Bildgebungswandler einen distalen Abschnitt und einen Zwischenabschnitt in der Nähe des distalen Abschnitts aufweist, wobei der distale Abschnitt einen ersten Durchmesser aufweist, der größer als ein zweiter Durchmesser des Zwischenabschnitts ist.

10. Ultraschalltherapiesystem nach Anspruch 9, wobei der transrektale Bildgebungswandler ferner einen proximalen Abschnitt nahe dem Zwischenabschnitt umfasst, wobei der proximale Abschnitt einen dritten Durchmesser aufweist, der größer als der zweite Durchmesser des Zwischenabschnitts ist.

11. Ultraschalltherapiesystem nach Anspruch 9, wobei der erste Durchmesser kleiner oder gleich 20 mm ist.

12. Ultraschalltherapiesystem nach Anspruch 9, wobei der zweite Durchmesser kleiner oder gleich 12 mm ist.

13. Ultraschalltherapiesystem nach Anspruch 9, wobei der erste Durchmesser im Bereich von 15 mm bis 20 mm und der zweite Durchmesser im Bereich von 6 mm bis 12 mm liegt.

14. Ultraschalltherapiesystem nach Anspruch 10, wobei der erste und dritte Durchmesser im Bereich von 15 mm bis 20 mm und der zweite Durchmesser im Bereich von 6 mm bis 12 mm liegen.

## Revendications

1. Système de thérapie par ultrasons destiné au traitement d'une prostate en passant par la fenêtre périnéale d'un patient, comprenant :
un transducteur de thérapie par ultrasons (800) dimensionné et conçu pour être positionné de manière adjacente au périnée du patient et générer et focaliser des impulsions ultrasonores sur la prostate, le transducteur de thérapie par ultrasons comprenant une ouverture (808) disposée le long ou près d'un périmètre du transducteur de thérapie par ultrasons ; et
un transducteur d'imagerie transrectal (802) disposé dans l'ouverture du transducteur de thérapie par ultrasons,
**caractérisé en ce que** :
l'ouverture du transducteur de thérapie par ultrasons permet des mouvements linéaires, panoramiques et d'inclinaison du transducteur de thérapie par ultrasons par rapport au transducteur d'imagerie transrectal sans contact ou interférence de la part du transducteur d'imagerie transrectal.

2. Système de thérapie par ultrasons selon la revendication 1, dans lequel le transducteur de thérapie présente un diamètre allant de 80 mm à 180 mm, une distance focale allant de 70 mm à 150 mm et un nombre F inférieur à 1,2.

3. Système de thérapie par ultrasons selon la revendication 1, dans lequel l'ouverture du transducteur de thérapie par ultrasons présente une forme choisie dans le groupe constitué par une forme ronde, une forme semi-circulaire, une forme carrée, une forme rectangulaire, une forme triangulaire et une forme plate.

4. Système de thérapie par ultrasons selon la revendication 1, dans lequel le transducteur de thérapie par ultrasons comprend un transducteur « en forme de coeur », le transducteur de thérapie par ultrasons ayant une extrémité large conçue pour être placée sur une partie postérieure du périnée et une extrémité étroite conçue pour être placée sur une partie antérieure du périnée, l'ouverture du transducteur de thérapie par ultrasons étant disposée sur l'extrémité large du transducteur de thérapie par ultrasons.

5. Système de thérapie par ultrasons selon la revendication 1, dans lequel le transducteur de thérapie par ultrasons comprend un seul élément de transducteur à ultrasons.

6. Système de thérapie par ultrasons selon la revendication 1, dans lequel le transducteur de thérapie par ultrasons comprend une pluralité d'éléments de transducteur à ultrasons.

7. Système de thérapie par ultrasons selon la revendication 6, dans lequel la pluralité d'éléments de transducteur à ultrasons sont ronds.

8. Système de thérapie par ultrasons selon la revendication 6, dans lequel la pluralité d'éléments de transducteur à ultrasons sont de type hexagonal.

9. Système de thérapie par ultrasons selon la revendication 1, dans lequel le transducteur d'imagerie transrectal comprend une section distale et une section intermédiaire à proximité de la section distale, la section distale présentant un premier diamètre plus grand qu'un deuxième diamètre de la section intermédiaire.

10. Système de thérapie par ultrasons selon la revendication 9, dans lequel le transducteur d'imagerie transrectal comprend en outre une section proximale à proximité de la section intermédiaire, la section proximale ayant un troisième diamètre plus grand que le deuxième diamètre de la section intermédiaire.

11. Système de thérapie par ultrasons selon la revendication 9, dans lequel le premier diamètre est inférieur ou égal à 20 mm.

12. Système de thérapie par ultrasons selon la revendication 9, dans lequel le deuxième diamètre est inférieur ou égal à 12 mm.

13. Système de thérapie par ultrasons selon la revendication 9, dans lequel le premier diamètre va de 15 mm à 20 mm et le deuxième diamètre va de 6 mm à 12 mm.

14. Système de thérapie par ultrasons selon la revendication 10, dans lequel les premier et troisième diamètres vont de 15 mm à 20 mm et le deuxième diamètre va de 6 mm à 12 mm.
